# EUROPEAN PATENT APPLICATION

(11) **EP 1 504 748 A1**
(43) Date of publication of application: **09.02.2005**
(21) Application number: 03018076.4
(22) Date of filing: 08.08.2003
(51) Int. Cl.: A61K 7/075

(54) **Hair conditioning composition**

(71) Applicant: KPSS-Kao Professional Salon Services GmbH, 64297 Darmstadt (DE)
(72) Inventor: Bräutigam, Ina, 64297 Darmstadt (DE)

(57) **Abstract**

The present invention relates to a gel type of hair conditioner composition showing relatively low viscosity changes at fluctuating temperatures and comprising at least one cationic polymer as a thickener and stabilizer and a silicone oil mixture and free from fatty alcohols, and, therefore, not in the emulsion form which is especially excellently suitable for rinse off application as well as leave in application. The conditioner composition of the present invention further comprises at least one solubilizer selected from non-ionic, cationic and/or amphoteric surfactants. Furthermore, the conditioners of the present invention include active ingredients selected from UV filters, antidandruff ingredients, hair restructuring ingredients, moisturising ingredients, sequestering agents and natural plant extracts showing hair conditioning and/or restructuring benefits. Moreover, the conditioners of the present invention can also optionally contain cationic direct dyes for coloring and/or color enhancing.

## Description

The present invention relates to a gel type of hair conditioner composition showing relatively low viscosity changes at fluctuating temperatures and comprising at least one cationic polymer as a thickener and stabilizer and a silicone oil mixture and free from fatty alcohols, and, therefore, not in the emulsion form which is especially excellently suitable for rinse off application as well as leave in application. The conditioner composition of the present invention further comprises at least one solubilizer selected from non-ionic, cationic and/or amphoteric surfactants. Furthermore, the conditioners of the present invention include active ingredients selected from UV filters, antidandruff ingredients, hair restructuring ingredients, moisturising ingredients, sequestering agents and natural plant extracts showing hair conditioning and/or restructuring benefits. Moreover, the conditioners of the present invention can also optionally contain cationic direct dyes for coloring and/or color enhancing.

Hair conditioners of various types either in application or in the form of preparation types have found their wide range of usage in hair dressing practice. Among those rinse off types of conditioners have always been preferred in hair care. Usually rinse off type of conditioners are in the form of emulsions and comprising fatty alcohols and emulsifiers of different character as the main principal ingredients. In addition, they certainly comprise conditioning ingredients of various types and those of common ingredients in cosmetic preparations. A general overview on the known hair conditioning products and also their usual compositions can be found in the monography of K. Schrader, "Grundlage und Rezepturen der Kozmetika", 2^{nd} Ed. 1989, pp 722 - 781.

Gel type of preparations are usually found not to be suitable for rinse off applications as after rinsing off, the hair is usually not combable, has dull appearance and is not manageable at all.

The cationic polymer used in the compositions of the present invention is known as Polyquaternium 37 with its CTFA adopted name. Chemically, it is homopolymer of dimethylaminoethyl methacrylate quarternized with methyl chloride. The same structure is available from Ciba Chemicals under the trade name Salcare SC95 and 96. However those two materials are delivered in suspension form either as dispersed in an oily medium or in a non-ionic surfactant mixture. Recently, from Cosmetic Rheologies Ltd., it has been possible to receive the polymer in a powder form which, first of all, do not contain any additional raw materials and dissolves easily in water and forms gel. It is marketed with the trade name Ultragel 300.

Unlike the other polymers known with the same CTFA adopted name, this is nearly pure raw material in powder form, it may certainly contain some impurities that do not disturb preparation of the conditioners of the present invention. The others, Salcare SC95 and 96 are both in a mixture with additional ingredients. The conditioners prepared with those do not show good hair conditioning properties when used as a rinse off conditioner. In other words, opposite to the ones prepared with the powder Polyquaternium 37, those conditioners thickened with Salcare types do not improve hair quality in terms of combability, elasticity, manageability, volume and body and shine when used as a rinse off conditioner.

The use of silicones in hair care compositions is as well known in leave in and rinse off preparations. The silicone oil used in the present inventions is known with the trade name Dow Corning 1503 and is a mixture of dimethicone and dimethiconol. The both silicones have been either alone or in combination have also found its application in hair care. However those have always been either in emulsion form or in another type such as two phase compositions. There has not been any composition available that is not in emulsion form. Furthermore, the literature is silent on the use of the combination of polyquaternium 37 and the mixture of dimethicone and dimethiconol.

The problem of using single composition as a rinse off and as well leave in preparation is being brought forward by this inventions. The present invention therefore concerns a composition usable as a rinse off and as well as leave in conditioner for achieving excellent hair qualities in terms of combability, softness, smoothness, volume body and especially shine. Another solution is being offered is that by combining the cationic polymer and silicone oil mixture it has also been achieved to use especially the silicone oil mixture at lower concentrations than that of currently being used. This brings certainly economic advantage as well.

Conditioner composition of present invention comprises a cationic polymer as the main thickener and stabilizer and a mixture of dimethicone and dimethiconol, does not contain any fatty alcohol and therefore is not in emulsion form. The conditioner has a consistency which is less sensitive to temperature fluctuations and furthermore especially excellently suitable for rinse off applications as well as leave in. The conditioner preparations of the present invention further comprises at least one solubilizer selected from non-ionic, cationic and/or amphoteric or zwitterionic surfactants. Furthermore, the conditioners of the present invention can include active ingredients selected from UV filters, antidandruff ingredients, hair restructuring ingredients, moisturising ingredients, sequestering agents and natural plant extracts showing hair conditioning and/or restructuring benefits. Cationic direct dyes can be added as well for hair colouring and colour enhancing purposes.

The conditioners of the present invention make hair easy to comb and improve shine elasticity, volume and body and particularly improves structure of those damaged, chemically processed hair, which is known under the concept "hair repair".

The conditioners of the present invention contain polyquaternium 37 known with the trade name Ultragel 300 at a concentration of 0.01 to 5%, preferably, 0.01 to 3.5% and more preferably 0.05 - 2.5% by weight calculated to the total composition.

The silicone oil is mixture of dimethicone and dimethiconol and dimethiconol is in the mixture at around 12%. So that the silicone oil mixture used in the present invention consists of around 88% dimethicone and around 12% dimethiconol.

It should be pointed out here that the ratio of the polyquaternium 37 to silicone oil mixture has been found important as well for stability. It has been found out that for the best performance and stability the ratio should be in the range of 1:1 to 1:20, preferably 1:1 to 1:15 and more preferably 1:1 to 1:10.

The conditioners of the present invention contain dimethicone/dimethiconol mixture known with the trade name Dow Corning 1503 at a concentration of 0.01 to 15%, preferably, 0.01 to 10% and more preferably 0.05 - 7.5%, most preferably 0.05 - 5% by weight calculated to the total composition

The conditioners of the present invention comprise in addition to the cationic polymer one or more cationic surfactants as conditioner and solubilzers presented with the general formula 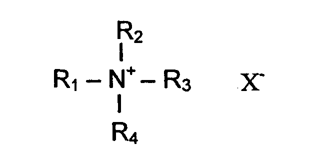 where R₁ is a saturated or unsaturated, branched or non-branched alkyl chain with 8-22 C atoms or

R₅ CO NH (CH₂)ₙ

where R₅ is saturated or unsaturated, branched or non-branched alkyl chain with 7-21 C atoms and n has value of 1 ~ 4 or

R₆ CO O (CH₂)ₙ

where R₆ is saturated or unsaturated, branched or non-branched alkyl chain with 7-21 C atoms and n has value of 1 ~ 4, and
R₂ is H or unsaturated or saturated, branched or non-branched alkyl chain with 1 ~ 4 C atoms or

R₅ CO NH (CH₂)ₙ

or

R₆ CO O (CH₂)ₙ

where R₅ , R₆ and n are same as above.
R₅ and R₆ are H or lower alkyl chain with 1 to 4 Carbon atoms, and X is chloride, bromide, methosulfate.

Typical examples of those ingredients are cetyl trimethly ammonium chloride, stear trimonium chloride, behentrimoinium chloride, stearamidopropyl trimonuim chloride, dioleoylethyl dimethyl ammonium methosulfate, dioleoylethyl hydroxyethylmonium methosulfate.
The concentration of cationic surfactants typically is from 0.01 to 10%, preferably 0.01 - 5%, more preferably 0.05 - 5% and most preferably 0.05 - 3.5% by weight, calculated to the total composition.

Furthermore, conditioners of the present invention comprise one or more non-ionic surfactants as solubilizers. Suitable nonionic surfactants are compounds from the category of alkyl polyglucosides with the general formula

R₇-O-(CH₂CH₂O)ₙ-Zₓ,

wherein R₇ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Further nonionic surfactants useful in the compositions according to invention are C₁₀-C₂₂-fatty alcohol ethoxylates. Especially suited C₁₀-C₂₂-fatty alcohol ethers are the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Other additionally useful surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R}".

Hydrogenated castor oil with variable ethylene glycol units are found to be suitable non-ionic solubilizers. Those are for example known from BASF under the trade name Cremophor.

Further additionally useful surfactants are amineoxides.
Such amineoxides are state of the art, for example C₁₂-C₁₈- alkyl dimethyl amineoxides such as lauryl dimethyl amineoxide, C₁₂-C₁₈- alkyl amidopropyl or ethyl amineoxides, C₁₂-C₁₈ -alkyl di(hydroxyethyl) or (hydroxypropyl) amineoxides, or also amineoxides with ethylene oxide and/or propylene oxide groups in the alkyl chain.

Suitable amineoxides are on the market, for example, under the trade names "Ammonyx®", "Aromox®" or "Genaminox®".

The concentration of nonionic surfactants is typically from 0.01 to 10%, preferably 0.05 - 7.5%, more preferably 0.1 - 5% and most preferably 0.1 - 3.5% by weight, calculated to the total composition.

Further optional surfactant components are fatty acid mono- and dialkanolamides, such as coco fatty acid monoethanolamide and myristic fatty acid monoisopropanolamide.

Conditioners of the present invention can contain amphoteric or zwitterionic surfactants as solubilizing agents. Suitable ones are in particular the various known betaines such as fatty acid amidoalkyl betaines and sulfobetaines; for example lauryl hydroxy sulfobetaine; long-chain alkyl amino acids, such as cocoaminoacetate, cocoaminopropionate and sodium cocoamphopropionate and -acetate have also proven suitable.

In detail it is possible to use betaines of the structure 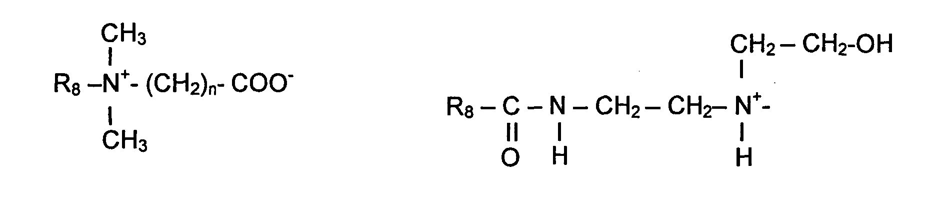 wherein R₈ is a C₈-C₁₈-alkyl group and n is 1 to 3,
sulfobetaines of the structure 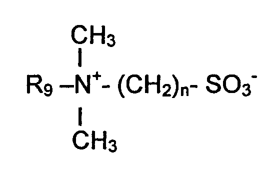 wherein R₉ is a C₈-C₁₈-alkyl group and n is 1 to 3,
and amidoalkyl betaines of the structure 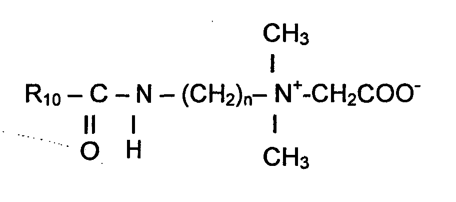 wherein R₁₀ is a C₈-C₁₈-alkyl group and n is 1 to 3,

Preferred are fatty acid amidoalkyl betaines, in particular cocoamidopropyl betaine, and cocoamphoacetate and -propionate, in particular the sodium salts thereof. The concentration can be typically less than 5%, preferably less than 3.5% by weight calculated to the total composition.

Solubilizers of nonionic, cationic and amphoteric character can certainly be used in combination in the conditioners of the present invention. In this case, the total concentration of the solubilizer in the conditioner should, as a rule, not exceed 15%, prefereably 10% and more preferably 7.5% by weight calculated to the total composition. It should be noted that the solubilizers preferred are nonionic and cationic surfactants.

Cosmetic compositions of the present invention can contain additional cationic polymers as conditioning agents. Suitable polymers are those of cationic polymers best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quatemium-8, Quaternium-14, Quatemium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84. It has been found out that Quatemium-80 is the best suitable one among the Quaterniums.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quatemized products of graft polymers from organopolysiloxanes and polyethyl oxazolines described in EP-A 524 612 and EP-A 640 643.

The hair cosmetic compositions of the present invention can further comprise silicone-conditioning agents. Those can be volatile as well non volatile ones, as well as nonionic and cationic ones are found to be suitable. It should be noted that for the selection compatibility should be evaluated first. Typical examples of those suitable ones are of Dow Corning^{R} series, as well phenyldimethicone, cyclomethicone, polydimethylsiloxane, polydiethylsiloxane, polymethylphenyl siloxane are found to be suitable. As a cationic silicone derivative, amodimethicone is found to be effective.

Typical concentration range for any of the conditioners mentioned above can be 0.01 - 5% by weight, preferably 0.01 - 3.5% by weight and more preferably 0.05 - 2% by weight calculated to the total composition.

The conditioner compositions may contain organic solvents, for example, as penetration enhancer such as ethanol. propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylene glycol, butylenes glycol, propylene glycol, benzyl glycol, ethylene glycol monoethylether, ethylene glycol monobutyl ether, ethylene glycol monophenyl ether, 1-phenylethylalcohol, 2-phenylethylalcohol, o-methoxyphenol. Concentration of organic solvents in the conditioner composition can be in the range from 0.1 to 10% by weight, preferably 0.1 to 7.5% by weight, and more preferably 0.1 to 5% by weight calculated to the total composition. The organic solvents may at the same time serve to solubilize ingredients which are not readily soluble in the conditioner composition.

Conditioner compositions of the present invention can optionally contain non-ionic polymers such as hydroxyethylcellulose, hydroxypropylclellulose, xanthan gum, xyloglucan, polyvinylalcohol, polyvinylpyrrolidone or their derivatives. Those may as well serve for thickening though for the best consistency performance, the concentration should be kept as low as possible and in any event should not exceed 25% by weight of the main thickener that is as well conditioner.

Anionic polymers should not be used in the conditioner compositions of the present invention as incompatibilities arose with the main thickening cationic polymer and other cationic conditioners. Typical example of those should not be used is acrylate type of polymers know with the trade name Carbopol from Goodrich.

The conditioner compositions can have viscosity values between 1,000 mPa.s to 70,000 mPa.s, preferably 1,000 mPa.s to 50,000 mPa.s and more preferably 1,000 mPa.s to 40,000 mPa.s measured at 20°C with Brookfield viscosimeter with, for example, Spindle 5 at 5 rpm. The viscosity values are read after 30 seconds from the start of the measurement. In the selection of the viscosity, special attention must be paid to the way of application and packaging to be used. It should be noted that the viscosity of the conditioners is less sensitive to temperature fluctuations. In other words, the viscosity changes observed with either decreasing or increasing temperatures is relatively small when compared to well known gel type of preparations thickened such as with hydroxyethylcellulose.

The pH of the conditioners of the present invention varies from 2 to 7, particularly 2 to 6 and more particularly 2.5 to 5.5. Moreover, the optimum conditioning properties observed with the compositions having pH at 4.0±0.5.

For adjusting the pH of the said conditioner compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be adjusted in a way that conditioner composition so obtained has a pH value between 2 to 7. Typically concentration for acids can be 0.01 - 5% by weight, preferably 0.01 - 4% by weight, more preferably 0.05 - 2.5% by weight calculated to the total composition. The pH of the conditioner composition can also be adjusted to the required pH by using alkaline solution such as sodium hydroxide, potassium hydroxide or their salts with those acids mentioned above in the case that at the selected acid concentration pH of the composition is lower than that of the aimed value.

The conditioner composition may contain active ingredients selected from UV filters, moisturisers, sequestering agents, antidandruff agents, hair restructuring agents and natural ingredients showing conditioning benefits. As well cationic direct dye serve to achieve excellent colour and colour enhancing effects on hair.

The UV filters useful in conditioners according to the present invention can be those of oil soluble, non-ionic, ones as well those of water soluble and mainly of anionic character. Examples are Benzophenone-1 Benzophenone-2, Benzophenone-3, Benzophenone-7, Benzophenone-6, Benzophenone-8, octylmethoxy cinnamate, homosalat to those of oil soluble ones and Benzophenone-4, benzophenone-9 to those anionic water soluble ones. It should be noted that the other UV filters of oil and water soluble ones should as well be possible to combine.

The moisturising agents are selected from panthenol, polyols, such as glycerol, polyethylene glycols with molecular weight 200 to 20,000. The moisturising ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

Antidandruff agent is preferably piroctone olamine (Octopirox). Other antidandruff agents such as climbazol can also be used as long as those are soluble in the conditioner compositions or can be solubilized with the aid of the solublizers mentioned above. Typical useful concentration range for sequestering agents is between 0.2 -1 % by weight calculated to the total composition.

Hair restructuring agents preferred are especially the ones disclosed in the German patent DE 197 51 550 C2.

One of the known hair restructuring agents is ceramide type of compound with the general formula 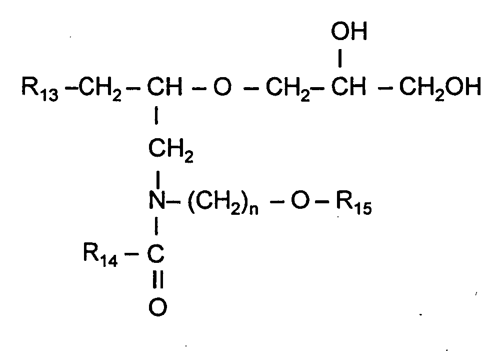 where R¹³ and R¹⁴ are independent from each other alkyl- or. alkenyl group mit 10 to 22 carbon atoms, R¹⁵ is methyl, ethyl, n-propyl or isopropyl group and n is a number between 1 to 6, preferably 2 or 3.

Other preferred hair restructuring agents are fatty acids with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the conditioners of the present invention can be in the range of 0.01 to 2 and especially 0.01 to 1% by weight calculated to the total weight of the composition. The fatty acids may be contained at a level of 0.01 to 2.5% and epecially 0.01 to 1% by weight calculated to the total weight of the composition. Phytosterol concentration of the conditioners is less than 1% and preferably less than 0.5% by weight calculated to the total weight of the composition. It should be noted that the combination of those three ingredients are preferably used in combination in the composition of the present invention. Special attention has to be paid when formulating such compositions to the transparency of the compositions. This can be achieved by carefully adjusting the solubilizers concentration in the compositions.

Natural plant extracts showing hair conditioning and/or restructuring effects can be used in the conditioners. Those are preferably the extracts from almond, Aloe Vera, coconut, mango, peach, lemon, wheat, rosemary, apricot, algae, grapefruit, sandalwood, lime and orange. Those extracts used are the ones commercially available and generally include organic solvents such as propylene glycol, butylenes glycol, ethanol, isopropanol. The active matter in those extracts can vary largely, i.e. in the range of 1 - 30% by weight. Concentration of those as an extract depending on the compatibility with the other ingredients can be in the range of 0.1 to 5%, preferably 0.1 to 2% by weight calculated to the total composition.

### Useful cationic direct dyes are:

Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 22, Basic Red 76, Basic Violet 1, Basic Violet 3, Basic Violet 10, Basic Violet 14, Basic Yellow 57, HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No. 14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15. Concentration of cationic dyes can typically be from 0.0001 to 2.5% preferavly from 0.0001 to 1% by weight calculated to the total composition.

The following examples are to illustrate the invention, but not limiting it.

### Example 1

| | |
|---|---|
| Ultragel 300 | 1.00 |
| Dimethicone/Dimethiconol* | 1.00 |
| Merquat 550 | 0.50 |
| Panthenol | 0.50 |
| Hydroxyethylcellulose | 0.20 |
| Glycerin | 2.00 |
| Cetrimonium chloride | 1.00 |
| Benzophenone-3 | 0.30 |
| Lactic acid | 0.07 |
| Fragrance | q.s. |
| Preservative | q.s. |
| Water | to 100 |

| | |
|---|---|
| * Dow Corning 1503 | |

Conditioner is prepared by dissolving Ultragel 300 in water first and other ingredients are added one by one to the gel solution. In the case of the solid ingredient it is preferable either alone or combined with others to prepare their solution first in a small portion of water. The conditioner thus obtained has a pH value of 4 and has a viscosity of around 17,000 mPa.s measured with Brookfield viscosimeter at 20°C with spindle 4 at 10 rpm. The conditioner so obtained is transparent.

For illustration of the insensitivity of the viscosity of the composition to the temperature fluctuations, viscosity of the above example is measured at various temperature. At the same time, a new composition is produced in which only hydroxyethylcellulose is used as thickener, so that one of the compositions produced is containing 1.2% hydroxyethylcellulose (HEC-1.2%). In order to illustrate that the viscosity is in the same range as in the inventive composition, the hydroxyethylcellulose concentration is increased to 1.7% by weight calculated to the total composition (HEC-1.7%). The viscosity results are presented in Table I.

**Table I**

| **Viscosity mPa.s** | | | |
|---|---|---|---|
| Temperature °C | Example 1 | HEC-1.2% | HEC-1.7% |
| 5 | 17500 | 6719 | 18720 |
| 20 | 17000 | 3720 | 13500 |
| 35 | 15900 | 2160 | 7500 |
| 50 | 14900 | 1240 | 4760 |

All viscosity values given above are measured with Brookfiled viscosimeter at given temperature, with spindle 4 and at 10 rpm.

As obvious from the results in Table I, viscosity of the conditioners thickened with hydroxyethylcellulose is strongly dependent on temperature whereas the viscosity of the inventive composition thickened with Polyquaternium 37 (Ultragel 300) changes slightly.

In order to show the conditioning effect of the inventive composition in rinse off application, the composition of example 1 is tested against a composition of the following formula. In the comparative example, polyquatemium 37 and dimethicon/dimethiconol is excluded, the concetration of Hydroxyethylcellulose is increased to 1.7% to thicken, and as a conditioner Merquat 550 concentration is increased to 14.5% raw material which corresponds to 1.16% by weight cationic conditioning ingredient. In addition cyclomethicone (Dow Corning 344) is added at 1% concentration (equal to the concentration of dimethicon/dimethiconol).

### Example 2 (comparative formula, not inventive)

| | |
|---|---|
| Merquat 550 | 14.50 |
| Cyclomethicone* | 1.00 |
| Panthenol | 0.50 |
| Hydroxyethylcellulose | 1.70 |
| Glycerin | 2.00 |
| Cetrimonium chloride | 1.00 |
| Lactic acid | 0.07 |
| Fragrance | q.s. |
| Preservative | q.s. |
| Water | to 100 |

| | |
|---|---|
| * Dow Coming 344 | |

Both compositions (inventive and comparative) are tested against each other in a half-head test on 10 women. For this purpose whole head of test person is washed with a Shine and Vitality shampoo (commercially available product under the brand name Goldwell Definition) and the equal amount from both conditioners are applied to each side and processed for 5 min. Subsequently rinsed off and towel dried and further dried with a dryer. In wet and dry stage sensory evaluations are made on the hair properties given in Table II.

**Table II:**

| **Sensory evaluation** | | | | |
|---|---|---|---|---|
| | | Prefered | | |
| | Parameter | Example 1 | Example 2 | no preference |
| Wet | Easy combing | 7 | 2 | 1 |
| | Smoothness | 6 | 2 | 2 |
| | Roughness | 2 | 3 | 5 |
| | | | | |
| Dry | Easy combing | 6 | 2 | 2 |
| | Smoothness | 6 | 1 | 3 |
| | Elasticity | 8 | 2 | 0 |
| | Volume | 4 | 5 | 1 |
| | Shine | 8 | 2 | 0 |

Similar results are obtained with the following inventive examples.

### Example 3

| | |
|---|---|
| Ultragel 300 | 1.20 |
| Dimethicone/dimethiconol* | 2.00 |
| Panthenol | 0.50 |
| Decyl polyglucoside | 3.50 |
| Ceramide of the formula** | 0.20 |
| Palmitic acid | 0.10 |
| Avocadin | 0.05 |
| Benzyloxyethanol | 2.00 |
| Glycerin | 2.00 |
| Behentrimoniumchloride | 2.00 |
| Octylmethoxycinnamate | 0.50 |
| Citric acid | q.s. to pH 4.5 |
| Fragrance | q.s. |
| Preservative | q.s. |
| Water | to 100 |

| | |
|---|---|
| *Dow Corning 1503 | |
| ** as given in the description R₁₃ is C₁₅H₃₁; R₁₄ is C₁₆H₃₃ and R₁₅ are H and n=2 | |

Formulation is prepared as described for example 1 except ceramide and palmitic acid are first dissolved in decyl polyglucoside and than added to the rest of the composition. The composition so obtained is clear and has a viscosity around 19,500 mPa.s measured with Brookfield viscosimeter at 20°C with spindle 4 at 10 rpm. The composition is especially suitable for damaged hair as restructurant, repair treatment.

### Example 4

| | |
|---|---|
| Ultragel 300 | 1.20 |
| Dimethicone/Dimethiconol* | 3.00 |
| Panthenol | 0.70 |
| PEG-60 hydrogenated castor oil | 0.50 |
| Aloe vera extract | 1.00 |
| Polyethylene glycol 200 | 1.00 |
| Stearamidopropylamine | 1.00 |
| Homosalat | 0.50 |
| Citric acid | q.s. to pH 3.5 |
| Fragrance | q.s. |
| Preservative | q.s. |
| Water | to 100 |

| | |
|---|---|
| *Dow Coming 1503 | |

Formulation is prepared as described for example 1 The composition so obtained has a viscosity around 23,000 mPa.s measured with Brookfield viscosimeter at 20°C with spindle 4 at 10 rpm. The composition is especially suitable for dry hair as a moisturising conditioner, either as rinse off or leave in.

### Example 5

| **Colour enhancing conditioner** | |
|---|---|
| Ultragel 300 | 1.20 |
| Dimethicone/Dimethiconol* | 3.00 |
| Panthenol | 0.70 |
| PEG-60 hydrogenated castor oil | 0.50 |
| Aloe vera extract | 1.00 |
| Polyethylene glycol 200 | 1.00 |
| Stearamidopropylamine | 1.00 |
| Homosalat | 0.50 |
| Basic red 76 | 0.10 |
| HC Red No 3 | 0.08 |
| Citric acid | q.s. to pH 4.0 |
| Fragrance | q.s. |
| Preservative | q.s. |
| Water | to 100 |

| | |
|---|---|
| *Dow Corning 1503 | |

Formulation is prepared as described for example 1. The composition so obtained has a viscosity around 22,000 mPa.s measured with Brookfield viscosimeter at 20°C with spindle 4 at 10 rpm. The composition is especially suitable for dry hair as a moisturising conditioner, either as rinse off or leave in. The compositions leaves a red shine on the hair. The shine is more perceivable in rinse off application than in leave-in

### Example 6

| **Gold Conditioner** | |
|---|---|
| Ultragel 300 | 1.20 |
| Dimethicone/Dimethiconol* | 3.00 |
| Panthenol | 0.70 |
| PEG-60 hydrogenated castor oil | 0.50 |
| Aloe vera extract | 1.00 |
| Polyethylene glycol 200 | 1.00 |
| Stearamidopropylamine | 1.00 |
| Benzophenone-3 | 0.10 |
| Basic yellow 57 | 0.008 |
| Basic brown 16 | 0.002 |
| Citric acid | q.s. to pH 4.0 |
| Fragrance | q.s. |
| Preservative | q.s. |
| Water | to 100 |

| | |
|---|---|
| *Dow Corning 1503 | |

Formulation is prepared as described for example 1. The composition so obtained has a viscosity around 22,500 mPa.s measured with Brookfield viscosimeter at 20°C with spindle 4 at 10 rpm. The composition is especially suitable for dry hair as a moisturising conditioner, either as rinse off or leave in. The compositions leaves a gold blond shine on the hair. The shine is more perceivable in rinse off application than in leave-in

## Claims

1. Hair conditioning composition **characterised in that** it comprises polyquaternium 37 as thickener and stabilizer and dimethicone/dimethiconol (88:12, weight ratio) mixture and free from fatty alcohols so that it is not in the form of an emulsion.

2. Hair conditioning composition according to claim 1 **characterised in that** it comprises polyquaternium 37 as thickener and stabilizer at a concentration between 0.01 to 5% by weight calculated to the total composition.

3. Hair conditioning composition according to claim 1 **characterised in that** it comprises dimethicone/dimethoconol (88:12, weight ratio) at a concentration between 0.01 to 15% by weight calculated to the total composition.

4. Hair conditioning composition according to claim 1 **characterised in that** it further comprises solubilizers selected from non-ionic, cationic and amphoteric/zwitterionic surfactants.

5. Hair conditioning composition according to any of the preceding claims **characterised in that** it further comprises active ingredients selected from UV filters, antidandruff ingredients, hair restructuring ingredients, moisturising ingredients, sequestering agents and natural plant extracts.

6. Hair conditioning composition according to any of the preceding claims **characterised in that** it comprises organic solvents as solubilizer and/or penetration enhancer.

7. Hair conditioning composition according to any of the preceding claims **characterised in that** it comprises as hair restructuring agents ceramide according to the formula 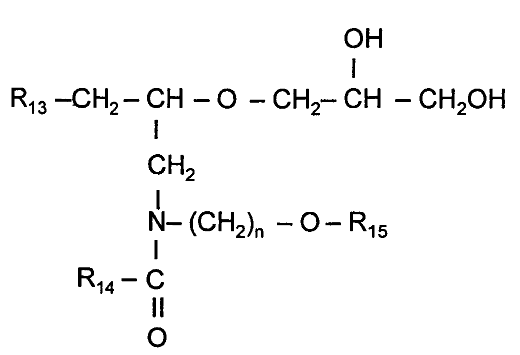 where R¹³ and R¹⁴ are independent from each other alkyl- or. alkenyl group mit 10 to 22 carbon atoms, R¹⁵ is methyl, ethyl, n-propyl or isopropyl group and n is a number between 1 to 6, preferably 2 or 3 and fatty acids with 10 to 24 carbon atoms and phytosterol.

8. Hair conditioning composition according to any of the preceding claims **characterised in that** it comprises cationic polymers as additional conditioners.

9. Hair conditioning composition according to any of the preceding claims **characterised in that** it comprises non-ionic polymers.

10. Hair conditioning composition according to any of the preceding claims **characterised in that** it is free from any of anionic polymers.

11. Hair conditioning composition according to any of the preceding claims **characterised in that** it comprises organic and/or inorganic acids and/or their mixtures and/or salts.

12. Hair conditioning composition according to any of the preceding claims **characterised in that** it comprises cationic direct dyes.

13. Hair conditioning composition according to any of the preceding claims **characterised in that** it has a pH in the range of 2 to 7.

14. Hair conditioning composition according to any of the preceding claims **characterised in that** it has a viscosity between 1,000 mPa.s to 70,000 mPa.s measured with Brookfield viscosimeter at 20°C with for example spindle 4 at 10 rpm.
